# EUROPÄISCHE PATENTSCHRIFT

(11) **EP 0 535 429 B1**
(45) Veröffentlichungstag und Bekanntmachung des Hinweises auf die Patenterteilung: **14.12.1994**
(21) Anmeldenummer: 92115652.7
(22) Anmeldetag: 14.09.1992
(51) Int. Cl.: A61K 7/06, A61K 7/11

(54) **Haarspray-Zusammensetzung**
Hair spray composition
Composition de laque capillaire

(30) Priorität: 02.10.1991 DE 4132776
(43) Veröffentlichungstag der Anmeldung: 07.04.1993
(73) Patentinhaber: GOLDWELL AKTIENGESELLSCHAFT, D-64297 Darmstadt (DE)
(72) Erfinder: Bünning, Einhard, W-6104 Seeheim-Jugenheim 1 (DE)

(56) Entgegenhaltungen:
- EP-A- 0 194 097
- EP-A- 0 242 792
- EP-A- 0 443 956
- CA-A- 1 002 256
- US-A- 3 728 447

## Beschreibung

Die vorliegende Erfindung betrifft eine neuartige Haarspray-Zusammensetzung, die zur Abgabe aus einer mechanischen Sprühvorrichtung oder mit verflüssigten Kohlenwasserstoff-Treibmitteln bzw. komprimierten Gasen vorgesehen ist und bei der Aufbringung auf das Haar die Frisur festigt, ihr einen elastischen Halt verleiht und zusätzlich den Haaren Glanz gibt.

Haarsprays, die mit Hilfe von verflüssigten Chlorfluorkohlenwasserstoff-Treibmitteln aus Aerosoldosen auf das Haar aufgebracht werden, sind seit langem bekannt.

Diese unter Druck verflüssigten Treibmittel wurden aufgrund ihrer vermuteten Umweltschädlichkeit bereits seit längerer Zeit durch Haarsprays abgelöst, die mit Hilfe von Kohlenwasserstoff-Treibmitteln, insbesondere Propan, Butan bzw. Isobutan und deren Gemischen oder komprimierter Gase, insbesondere Dimethylether, Kohlendioxid, Stickstoff oder Stickstoffoxid versprüht werden, oder durch aus mechanischen Sprühvorrichtungen abgegebene Haarspray-Zusammensetzungen ersetzt. Obwohl die heute eingesetzten Haarsprays bereits einen hohen Entwicklungsstand erreicht haben, erfüllen sie häufig doch nicht alle an sie gestellten Forderungen.

Ziel der Erfindung ist es daher, eine Haarspray-Zusammensetzung zur Abgabe aus einer mechanischen Sprühvorrichtung oder mit verflüssigten Kohlenwasserstoff-Treibmitteln bzw. komprimierten Gasen zu schaffen, die dem Haar eine hohe Festigung vermittelt, ohne gleichzeitig dieses starr bzw. hart zu verformen, sondern dem Haar ein flexibles Volumen gibt und darüber hinaus auch Glanz verleiht.

Diese Aufgabe wird dadurch gelöst, daß man eine Haarspray-Zusammensetzung auf wäßriger Basis, die etwa 30 bis 90 Gew.-%, insbesondere etwa 40 bis etwa 75 Gew Gew.-%, bezogen auf die Gesamtzusammensetzung, eines niedrigen Alkohols der Gruppe Ethylalkohol,n-Propylalkohol und/oder Isopropylalkohol und etwa 1 bis 15 Gew.-%, bezogen auf die Gesamtzusammensetzung, mindestens eines filmbildenden Polymeren enthält, noch etwa 0,1 bis etwa 5 Gew.-% mindestens eines C₆-C₁₈-Acyllactylats bzw. ein wasserlösliches Salz desselben zusetzt.

Die angegebenen Mengenanteile beziehen sich jeweils auf die Gesamtzusammensetzung ohne einen etwaigen Treibmittel-Anteil.

Acyllactylate der allgemeinen Struktur 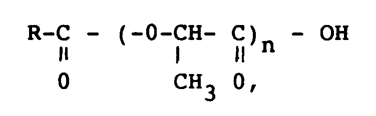
wobei R eine gerad- oder verzweigtkettige Alkyl- oder Alkenylgruppe mit 6 bis 18 Kohlenstoffatomen bedeutet, und n für eine Zahl zwischen 1 und 5 steht, und ihre Anwendung in kosmetischen Mitteln, insbesondere als Feuchthaltemittel, sind seit langem bekannt.

Es wird hierzu auf einen Artikel von L.J. Murphy und F. Baiocchi, Drug Cosmetic Industry 122/5 (May 1978), 35, "The Role of Acyl Lactylates in Cosmetics and Toiletries" verwiesen, wonach sie insbesondere als Feuchthaltemittel eingesetzt werden.

Aus der US-PS 3 275 503 ist die Verwendung dieser Substanzen, insbesondere von Natriumcaproyllactylat, als antimikrobielle Wirkstoffe beschrieben.

Aus der US-PS 3 472 940 ist weiterhin die Verwendung von Acyllactylaten, beispielsweise Natriumstearoyl-2-lactylat, in Deodorant-Stiften bekannt.

Die US-PS 3 728 447 schließlich beschreibt die Verwendung von Fettsäurelactylaten und -glycolaten in Konditionierungs-Shampoos.

Ebenso ist aus der EP-A 278 370 die Verwendung von verzweigtkettigen Acyllactylaten und deren Salzen in kosmetischen Mitteln, insbesondere auch Shampoos und Haarspülungen, bekannt.

Alle diese Druckschriften enthalten jedoch keinerlei Hinweis auf die überraschende Wirkung dieser Verbindungsklasse in Kombination mit filmbildenden Polymeren bei Anwendung in Haarspray-Zusammensetzungen zur Verbesserung der Elastizität und des Volumens sowie der Erhöhung des Glanzes des Haares.

Wie bereits ausgeführt, werden als im Rahmen der Erfindung geeignete Acyllactylate sowohl gerad- als auch verzweigtkettige Verbindungen eingesetzt. Besonders bevorzugt sind hierbei Stearoyl- und Isostearoyllactylat; die Herstellung des letzteren und anderer verzweigtkettiger Acyllactylate ist in der bereits erwähnten EP-A 278 370 beschrieben, auf die in diesem Zusammenhang ausdrücklich Bezug genommen wird.

Weitere geeignete Acyllactylate sind das Caproyllactylat und dessen Salze, Decanoyllactylat, Lauroyllactylat, Myristoyllactylat, etc. Die bevorzugte Zahl der Milchsäuregruppen liegt bei n = 1 bis 2, jedoch sind auch höhere Zahlen zwischen 3 und 5 möglich.

Die Zahl der Milchsäuregruppen im Acyllactylat wird durch die eingesetzten Mengenanteile und die Reaktionsbedingungen bestimmt; es wird hierzu auch auf die US-PS 2 733 252 verwiesen.

Eine Gruppe von Acyllactylaten, die zum Einsatz in den erfindungsgemäßen Haarsprays geeignet sind, werden von der Firma Patco Cosmetic Products, C.J. Patterson Co., unter der Bezeichnung "Pationic" vertrieben.

Wie bereits ausgeführt, können neben den Acyllactylaten selbst auch ihre wasserlöslichen Salze verwendet werden. Bevorzugt sind hierbei die Alkalisalze wie das Natrium-, Kalium- und Ammoniumsalz sowie Erdalkalisalze, beispielsweise Calciumacyllactylate; jedoch können auch Alkanolaminsalze zum Einsatz gelangen.

Auch ungesättigte Acyllactylate, wie sie z.B. in der EP-A 443 956 beschrieben sind, können im Rahmen der Erfindung verwendet werden.

Der bevorzugte Anteil an Acyllactylat liegt zwischen etwa 0,1 bis 5 Gew.-%, vorzugsweise 0,25 bis 2,5 Gew-%, insbesondere 0,5 bis 1,5 Gew.%, jeweils berechnet auf die Gesamtzusammensetzung.

Bei den erfindungsgemäß mit den Acyllactylaten bzw. deren wasserlöslichen Salzen zum Einsatz gelangenden filmbildenden Polymeren handelt es sich in der Regel um bekannte Produkte.

So sind die bekannten Vinylacetat/Vinylpyrrolidon-Copolymeren ebenso geeignet wie jene aus Acrylsäure/Vinylacetat, Terpolymerisate aus Vinylpyrrolidon, Acrylsäureestern und (Meth) Acrylsäure sowie Copolymerisate aus Vinylethern und Maleinsäureanhydrid, die in wässrigem Medium hydrolysieren, kationische Polymere, beispielsweise Copolymere aus Vinylpyrrolidon und Dialkylaminoalkyl(meth)acrylaten, die quaternisiert sein können, Homo- und Copolymere des Dimethyldiallylammoniumchlorids, beispielsweise mit Acrylamid, Copolymere aus Hydroxyethylcellulose und Diallyldimethylammoniumchlorid, mit Epichlorhydrin vernetzte Hydroxyethylcellulose, die anschließend mit Trimethylamin quaterniert wurde, natürliche Filmbildner wie Chitosan, etc.

Besonders bevorzugt als Filmbildner in den erfindungsgemäßen Haarspray-Zusammensetzungen wird ein Vinylpyrrolidon/Vinylacetat-Copolymer, beispielsweise ein solches unter dem Handelsnamen "LUVISKOL VA 28 " erhältliches in einer Menge von 1 bis 15 Gew.-%, vorzugsweise 2,5 bis 10 Gew.-%,insbesondere 5 bis 8 Gew.-%, berechnet auf die Gesamtzusammensetzung.

Gemäß einer weiteren bevorzugten Ausführungsform der Erfindung liegt dieses Vinylacetat/Vinylpyrrolidon-Copolymerisat im Gemisch mit einem kationischen Polymeren, beispielsweise einem quaternisierten Vinylpyrrolidon/Dialkylaminoalkylmethacrylat-Copolymerisat, vor, wobei der Anteil des kationischen Polymeren bei etwa 0,1 bis etwa 2,5 Gew.-% der Gesamtzusammensetzung, vorzugsweise zwischen etwa 0,5 und 1,5 Gew.-%, liegt.

Die erfindungsgemäßen Zusammensetzungen können alle in solchen Mitteln üblichen Stoffe enthalten, beispielsweise weitere Lösungsmittel wie niedere Alkane, beispielsweise n-Pentan oder Isopentan in Mengenanteilen zwischen etwa 2,5 und etwa 10 Gew.-% der Gesamtzusammensetzung, Aceton, Polyalkohole und deren Ether, beispielsweise Glycerin, Propylenglykol und dessen Monomethylether, Weichmacher, haarpflegende Wirkstoffe, wie beispielsweise Panthenol oder Phytantriol, Parfüms, oberflächenaktive Substanzen, etc.

Art und Menge solcher Zusatzstoffe sind dem Fachmann hinreichend bekannt und finden sich in jedem kosmetischen Handbuch.

Obwohl erfindungsgemäß aus mechanischen Sprühvorrichtungen abzugebende Haarsprays, sog. Pumpsprays, bevorzugt sind, kann auch mit komprimierten Gasen, vorzugsweise Dimethylether, Kohlendioxid, Stickstoff oder Stickstoffoxid (N₂0) oder Kohlenwasserstoff-Treibgasen wie Propan und Butan bzw. Isobutan und deren handelsüblichen Gemischen gearbeitet werden.

Im folgenden werden Ausführungsbeispiele für erfindungsgemäße Zusammensetzungen gegeben.

| Beispiel 1 | |
|---|---|
| Ethanol | 75,0 (Gew.-%) |
| Vinylacetat / Vinylpyrrolidon (80:20)-Copolymerisat | 7,0 |
| Quaterniertes Vinylpyrrolidon/Dimethylaminoethylmetharcylat-Copolymerisat | 0,5 |
| Dimethylsiloxan - Glycol-Copolymer (Dimethicone Copolyol) | 0,5 |
| Panthenol | 0,1 |
| Natriumisostearoyllactylat (n =1-2) | 0,5 |
| Parfum | 0,1 |
| Wasser | ad 100,0 |

Bei Anwendung aus einer bekannten mechanischen Pumpsprühvorrichtung auf das Haar wird eine Frisur mit vollem Griff, gutem Halt und natürlichem Glanz erhalten.

Die Weglassung des Natriumisostearoyllactylats in der gleichen Rezeptur ergab einen wesentlich starreren, harten Film auf dem Haar mit stumpfem Aussehen.

| Beispiel 2 | |
|---|---|
| Isopropylalkohol | 73,5 (Gew.-%) |
| Vinylacetat / Crotonsäure-Copolymerisat | 6,0 |
| Natriumstearoyllactylat (n = 2) | 1,0 |
| Phytantriol | 0,3 |
| Panthenol | 0,5 |
| Parfüm | 0,3 |
| Wasser | ad 100,0 |

Die Zusammensetzung wird als Pumpspray abgegeben.

| Beispiel 3 | |
|---|---|
| Ethanol | 51,5 (Gew.-%) |
| Vinylcaprolactam/Vinylpyrrolidon / Dimethylaminoethylmethacrylat -Copolymerisat | 7,2 |
| Vinylpyrrolidon/Vinylacetat -Copolymerisat | 1,5 |
| Natrium-n-caproyllactylat (n = 2) | 1,0 |
| Parfüm | 0,2 |
| Dimethylether | 25,0 |
| Wasser | ad 100,0 |

Die Abfüllung der Lösung mit Dimethylether in eine Aerosoldose erfolgt auf übliche Weise.

Es wird ein Haarspray erhalten, das dem Haar festen, aber elastischen Halt und Glanz verleiht.

| Beispiel 4 | |
|---|---|
| Ethanol | 50,50 (Gew.-%) |
| Copolymerisat aus Octylacrylamid/t-Butylaminoethylmethacrylat/Acrylsäure (Amphomer^{R}) | 4,50 |
| Quaterniertes Copolymerisat aus Vinylpyrrolidon/Dimethylaminooethylmethacrylat | 1,00 |
| Aminomethylpropanol | 0,75 |
| Dimethicone Copolyol | 0,50 |
| Natriumisostearoyllactylat (n = 1-2) | 1,50 |
| Parfüm | 0,15 |
| Wasser | 11,10 |
| abgefüllt mit einem Treibmittelgemisch Propan/Butan (35:65) | 30,00 |

## Patentansprüche

1. Haarspray-Zusammensetzung zur Abgabe aus einer mechanischen Sprühvorrichtung oder mit verflüssigten Kohlenwasserstoff-Treibmitteln bzw. komprimierten Gasen, enthaltend etwa 30 bis 90 Gew.-%, bezogen auf die Gesamtzusammensetzung, eines niedrigen Alkohols aus der Gruppe Ethylalkohol, n-Propylalkohol und/oder Isopropylalkohol, etwa 1 bis 15 Gew.-%, bezogen auf die Gesamtzusammensetzung, mindestens eines filmbildenden Polymeren sowie Wasser, dadurch gekennzeichnet, daß sie zusätzlich etwa 0,1 bis etwa 5 Gew.-% mindestens eines Acyllactylats der allgemeinen Formel 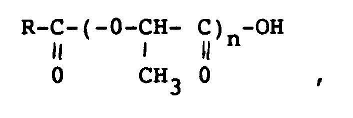 wobei R eine gerad- oder verzweigtkettige Alkyl- oder Alkenylgruppe mit 6 bis 18 Kohlenstoffatomen bedeutet, und n für eine Zahl zwischen 1 und 5 steht, oder ein wasserlösliches Salz desselben enthält.

2. Haarspray-Zusammensetzung nach Anspruch 1, dadurch gekennzeichnet, daß sie 0,25 bis 2,5 Gew.-%, berechnet auf die Gesamtzusammensetzung, eines Stearoyl-oder Isostearoyllactylats bzw. dessen Alkali- oder Ammoniumsalz enthält.

3. Haarspray-Zusammensetzung nach Anspruch 1 oder 2, dadurch gekennzeichnet, daß sie als filmbildendenes Polymeres 2,5 bis 10 Gew.-%, bezogen auf die Gesamtzusammensetzung, eines Vinylpyrrolidon/Vinylacetat-Copolymeren enthält.

4. Haarspray-Zusammensetzung nach Anspruch 3, dadurch gekennzeichnet, daß sie zusätzlich etwa 0,1 bis 2,5 Gew.-%, bezogen auf die Gesamtzusammensetzung, eines kationischen Polymeren enthält.

5. Haarspray-Zusammensetzung nach Anspruch 4, dadurch gekennzeichnet, daß sie als kationisches Polymer ein quaternisiertes Vinylpyrrolidon/Dialkylaminoethylmethacrylat-Copolymerisat enthält.

6. Verwendung eines Acyllactylats der allgemeinen Formel 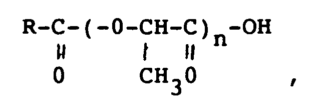 wobei R eine gerad- oder verzweigtkettige Alkyl- oder Alkenylgruppe mit 6 bis 18 Kohlenstoffatomen bedeutet, und n für eine Zahl zwischen 1 und 5 steht,
zur Herstellung von Haarspray-Zusammensetzungen auf wässriger Basis, die zur Abgabe aus einer mechanischen Sprühvorrichtung oder mit verflüssigten Kohlenwasserstoff-Treibmitteln bzw. komprimierten Gasen geeignet sind, und etwa 30 bis 90 Gew.-%., berechnet auf die Gesamtzusammensetzung, eines niedrigen Alkohols mit 2 bis 3 Kohlenstoffatomen, und etwa 1 bis 15 Gew.-%, berechnet auf die Gesamtzusammensetzung, mindestens eines filmbildenden Polymeren enthalten.

## Claims

1. Hair-spray composition for dispensing from a mechanical spraying device or with liquefied hydrocarbon propellents or compressed gases, resp., containing about 30 to 90% by weight, calculated to the total composition, of a lower alcohol selected from ethyl alcohol, n-propyl alcohol and (or) isopropyl alcohol, and about 1 to 10% by weight, calculated to the total composition, of at least one film-forming polymer and water, characterized in that it additionally contains about 0.1 to about 5% by wt. of at least one acyl lactylate of the general formula 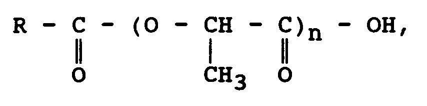 or a water-soluble salt thereof, wherein R is a straight- or branched-chain C₆-C₁₈-alkyl or alkenyl group, and n is a number between 1 and 5.

2. Hair-spray composition according to claim 1, characterized in that it contains 0.25 to 2.5% by weight, calculated to the total composition, of a stearoyl or isostearoyl lactylate or an alkali or ammonium salt thereof.

3. Hair-spray composition according to claim 1 or 2, characterized in that it contains 2.5 to 10% by weight, calculated to the total composition, of a vinyl pyrrolidone/vinyl acetate copolymer as a film-forming polymer.

4. Hair-spray composition according to claim 3, characterized in that it additionally contains 0.1 to 2.5% by weight, calculated to the total composition, of a cationic polymer.

5. Hair-spray composition according to claim 4, characterized in that it contains a quaternized copolymer of vinyl pyrrolidone and dialkyl aminoethyl methacrylate as cationic polymer.

6. Use of an acyl lactylate of the general formula 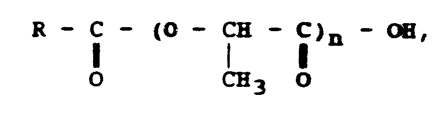 wherein R denotes a straight- or branched-chain C₆-C₁₈-alkyl or alkenyl group and n stands for a number between one and 5, for the production of water-based hair spray compositions which are suitable for dispensing from a mechanical spraying device or with liquefied hydrocarbon propellents or compressed gases comprising, calculated to the total composition, about 30 to 90% by weight of a lower alcohol having 2 to 3 carbon atoms and about 1 to 15% by wt., calculated to the total composition, of at least one film-forming polymer.

## Revendications

1. Composition de laque pour cheveux destinée à être distribuée à l'aide d'un dispositif mécanique de vaporisation ou par un agent de propulsion du type hydrocarbure liquéfié ou par des gaz comprimés, contenant environ de 30 à 90 % en poids par rapport à la composition totale, d'un alcool inférieur du groupe constitué par l'alcool éthylique, l'alcool n-propylique et/ou l'alcool isopropylique, environ 1 à 15% en poids par rapport à la composition totale d'au moins un polymère filmogène ainsi que de l'eau, caractérisé en ce qu'elle contient en outre environ 0,1 à environ 5% en poids d'au moins un acyllactylate de formule générale 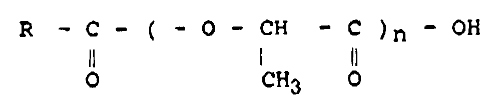 dans laquelle R représente un groupe alkyle ou alkényle linéaire ou ramifié ayant de 6 à 18 atomes de carbone, et n représente une nombre entre 1 et 5, ou d'un de ses sels hydrosolubles.

2. Composition de laque pour cheveux selon la revendication 1, caractérisée en ce qu'elle contient de 0,25 à 2,5% en poids par rapport à la composition totale d'un stéaroyl- ou d'un isostéaroyllactylate, ou d'un de leurs sels de métal alcalin ou d'ammonium.

3. Composition de laque pour cheveux selon l'une des revendications 1 ou 2, caractérisée en ce qu'elle contient comme polymère filmogène, de 2,5 à 10% en poids par rapport à la composition totale, d'un copolymère vinylpyrrolidone / acétate de vinyle.

4. Composition de laque pour cheveux selon la revendication 3, caractérisée en ce qu'elle contient en outre d'environ 0,1 à 2,5% en poids par rapport à la composition totale, d'un polymère cationique.

5. Composition de laque pour cheveux selon la revendication 4, caractérisée en ce qu'elle contient comme polymère cationique, un copolymérisat de vinylpyrrolidone / méthacrylate de dialkyleaminoéthyle quaternisé.

6. Utilisation d'un acyllactylate de formule générale 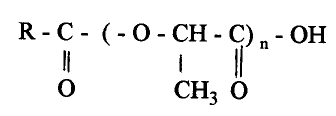 dans laquelle R représente un groupe alkyle ou alkényle linéaire ou ramifié de 6 à 18 atomes de carbone, et n représente une nombre entre 1 et 5, pour la préparation de compositions de laque pour cheveux à base aqueuse, destinées à être distribuées à l'aide d'un dispositif mécanique de vaporisation ou par un agent de propulsion du type hydrocarbure liquéfié ou par des gaz comprimés, et contenant environ 30 à 90 % en poids par rapport à la composition totale d'un alcool inférieur de 2 à 3 atomes de carbone, et environ 1 à 15 % en poids par rapport à la composition totale d'au moins un polymère filmogène.
